# EUROPEAN PATENT SPECIFICATION

(11) **EP 1 976 816 B1**
(45) Date of publication and mention of the grant of the patent: **14.04.2010**
(21) Application number: 06850588.2
(22) Date of filing: 18.12.2006
(51) Int. Cl.: C07C 43/11, C08G 65/26

(54) **BASE-CATALYZED ALKOXYLATION IN THE PRESENCE OF NON-LINEAR POLYOXYETHYLENE-CONTAINING COMPOUNDS**
BASENKATALYSIERTE ALKOXYLIERUNG IN GEGENWART NICHTLINEARER POLYOXYETHYLENHALTIGER VERBINDUNGEN
ALCOXYLATION CATALYSEE PAR UNE BASE EN PRESENCE DE COMPOSES NON LINEAIRES CONTENANT DU POLYOXYETHYLENE

(30) Priority: 22.12.2005 US 315639
(43) Date of publication of application: 08.10.2008
(73) Proprietor: Bayer MaterialScience LLC, Pittsburgh, PA 15205-9741 (US)
(72) Inventor: HAIDER, Karl, W., Wexford, PA 15090 (US); PAZOS, Jose, F., Charleston, WV 25304 (US); RODBERG, Steven, J., Charleston, WV 25304 (US)
(74) Representative: Klimiuk, Meike
(86) International application number: PCT/US2006/048184
(87) International publication number: WO 2007/117295

(56) References cited:
- MIHAIL IONESCU ET AL: "SYNTHESIS OF POLYETHER POLYOLS FOR FLEXIBLE FOAMS WITH COMPLEXED COUNTER-ION" CELLULAR POLYMERS. INTERNATIONAL CONFERENCE. BOOK OF PAPERS, XX, XX, vol. 4, 1997, pages 1-8, XP009085366

## Description

### FIELD OF THE INVENTION

The present invention relates in general to polyether polyols, and more specifically, to a long-chain polyether polyol having a number average molecular weight of more than about 1,200 g/mole and produced by alkoxylating an initiator in the presence of a basic catalyst having at least one cation thereof chelated with a non-linear polyoxyethylene-containing compound having a functionality of at least about three.

### BACKGROUND OF THE INVENTlON

It has been known for many years that cyclic ethers complex potassium ions strongly. Crown ethers were discovered in the 1960's by Charles Pederson and he was awarded the Nobel Prize in 1987 for his efforts. The ability of cyclic ethers to strongly complex metal ions led to much scientific work. Unfortunately, because of the synthetic difficulty, high cost and high toxicity of these compounds, crown ethers have never found wide commercial application. Perhaps because crown ethers were discovered first, most of those skilled in the art have overlooked the strong complexing abilities possessed by non-cyclic polyethers. Among the other advantages of non-cyclic polyethers are ready availability, low cost and the fact that polymers and oligomers of ethylene oxide are so non-toxic as to be acceptable food additives.

A commonly-assigned U.S. patent application filed on an even date herewith and entitled "Base-catalyzed alkoxylation in the presence of polyoxyethylene-containing compounds", (Atty. Docket No. PO8708, U.S. Serial No. 11/315,517) discloses a molecular weight dependency for a polyoxyethylene-containing additive which acts as a chelating agent in the base-catalyzed alkoxylation of long-chain polyethers.

A second commonly-assigned U.S. patent application also filed on an even date herewith and entitled "Short chain polyether polyols for rigid polyurethane foam", (Atty. Docket No. PO8707, U.S. Serial No. 11/315 513) discloses a polyoxyethylene-containing additive as a chelating agent in the alkoxylation of short chain polyethers.

Finally, a third commonly-assigned U.S. patent application also filed on an even date herewith and entitled 11/315 667) polyether polyols", (Atty. Docket No. PO8706, U.S. Serial No. 11/315 667) discloses a polyoxyethylene-containing initiator as a chelating agent in the alkoxylation of long-chain polyethers.

Although the concept of using linear polyoxyethylene compounds, like polyethylene glycols or "PEGs" for rate enhancement of the KOH catalyzed alkoxylation of long-chain polyols is known in the art (*See* "Synthesis of Polyether Polyols for Flexible Polyurethane Foams with Complexed Counterion" by Mihail lonescu. Viorica Zugravu, loana Mihatache and ion Vasile, Cellular Polymers IV. International Conference, 4th, Shrewsbury, UK, June 5-6, 1997. Paper 8, 1-8. Editor(s): Buist, J. M.), there are to our knowledge no published reports of using non-linear polyoxyethylene-containing compounds in the production of long chain polyether polyols, or of the effects on foams made with these non-linear polyoxyethylene-containing polyols.

It would therefore be desirable to provide long-chain polyether polyols produced by basic catalysis in the presence of non-linear polyoxyethylene-containing compounds and to demonstrate the utility of these polyols in making flexible polyurethane foams and non-cellular polyurethanes.

### SUMMARY OF THE INVENTION

Accordingly, the present invention provides a long-chain polyether polyol having a molecular weight of more than about 1,200 g/mole and produced by alkoxylating an initiator with an alkylene oxide in the presence of a basic catalyst having at least one cation thereof chelated with a polyoxyethylene-containing compound having a functionality of at least about three. The inventive polyols may be used to provide flexible polyurethane foams.

These and other advantages and benefits of the present invention will be apparent from the Detailed Description of the Invention herein below.

### DETAILED DESCRIPTION OF THE INVENTION

The present invention will now be described for purposes of illustration and not limitation. Except in the operating examples, or where otherwise indicated, all numbers expressing quantities, percentages, OH numbers, functionalities and so forth in the specification are to be understood as being modified in all instances by the term "about." Equivalent weights and molecular weights given herein are number average equivalent weights and number average molecular weights respectively, unless indicated otherwise.

The present invention provides a long-chain polyether polyol having a number average molecular weight of more than 1,200 g/mole and produced by alkoxylating an initiator with an alkylene oxide in the presence of a basic catalyst having at least one cation thereof chelated with a non-linear polyoxyethylene-containing compound having a functionality of at least about three.

The present invention further provides a process for producing a long chain polyether polyol having a number average molecular weight of more than 1,200 g/mole, the process Involving alkoxylating an initiator with an alkylene oxide in the presence of a basic catalyst having at least one cation thereof chelated with a non-linear polyoxyethylene-containing compound having a functionality of at least about three.

The present invention still further provides a flexible polyurethane foam made from the reaction product of at least one polyisocyanate and at least one long-chain polyether polyol having a number average molecular weight of more than 1,200 g/mole and produced by alkoxylating an initiator with an alkylene oxide in the presence of a basic catalyst having at least one cation thereof chelated with a non-linear polyoxyethylene-containing compound having a functionality of at least about three, optionally in the presence of at least one of blowing agents, surfactants, cross-linking agents, extending agents, pigments, flame retardants, catalysts and fillers.

The present Invention also provides a process for producing a flexible polyurethane foam involving reacting at least one polyisocyanate and at least one long-chain polyether polyol having a number average molecular weight of more than 1,200 g/mole and produced by alkoxylating an initiator with an alkylene oxide in the presence of a basic catalyst having at least one cation thereof chelated with a non-linear polyoxyethytene-containing compound having a functionality of at least about three, optionally in the presence of at least one of blowing agents, surfactants, cross-linking agents, extending agents, pigments, flame retardants, catalysts and fillers.

By "long-chain" polyether polyol, the inventors herein mean a polyether polyol having a number average molecular weight of greater than 1,200 g/mole, preferably from 1,200 to 50,000 g/mole, more preferably from 1,200 to 30,000 g/mole, and most preferably from 1,200 to 8,000 g/mole. The molecular weight of the inventive polyols may be in an amount ranging between any combination of these values, inclusive of the recited values.

The long chain polyether polyols of the present invention are made by basic catalysis, the general conditions of which are familiar to those skilled in the art. The basic catalyst may be any basic catalyst known in the art, more preferably the basic catalyst is one of potassium hydroxide, sodium hydroxide, barium hydroxide and cesium hydroxide, most preferably the basic catalyst is potassium hydroxide.

Suitable initiator (or starter) compounds include, but are not limited to, C₁-C₃₀ monols, ethylene glycol, diethylene glycol, triethylene glycol, propylene glycol, 1,3-propanediol, dipropylene glycol, tripropylene glycol, neopentyl glycol, 1,4-butanediol, 1,3-butanediol, 1,2-butanediol, 2.3-butanediol, 1,6-hexanediol, glycerin, trimethylolpropane, trimethylolethane, pentaerythritol, α-methylglucoside, sorbitol, mannitol, hydroxymethylglucoside, hydroxypropylglucoside, sucrose, N,N,N',N'-tetrakis[2-hydroxyethyl or 2-hydroxypropyl]ethylene diamine, 1,4-cyclohexanediol, cyclohexanedimethanol, hydroquinone, resorcinol, and the like. Nominal initiator functionality, which is understood to represent the ratio of the total number of equivalents of active hydrogens (as determined by the Zerewitinoff method) to moles in the starter mixture is from 2 to 8 or more, preferably from 2 to 6, and more preferably from 2 to 4. The functionality of the initiators useful in the present invention may be in an amount ranging between any combination of these values, inclusive of the recited valuers. Any mixtures of monomeric initiators or their oxyalkylated oligomers may also be utilized.

A non-linear polyoxyethylene-containing compound, such as an ethoxylated glycerine, is added to chelate at least one of the cations of the basic catalyst during the alkoxylation in the inventive long-chain polyether polyol production process. This non-linear polyoxyethylene-containing compound preferably has a functionality of at least three, more preferably from 3 to 8. The functionality of the non-linear polyoxyethylene-containing compound may be in an amount ranging between any combination of these values, inclusive of the recited values. The non-linear polyoxyethylene-containing compound preferably has a molecular weight of less than 10,000 g/mole and more preferably from 300 g/mole to 1,000 g/mole. The non-linear polyoxyethylene-containing compound may have a molecular weight in an amount ranging between any combination of these values, inclusive of the recited values.

The non-linear polyoxyethylene-containing compound is preferably added in an amount of from 0.5 to 20 wt.%, more preferably from 1 to 10 wt.%, and most preferably in an amount of from 2 to 9 wt.%, wherein the weight percentages are based on the weight of the long-chain polyether polyol. The non-linear polyoxyethylene-containing compound may be added in an amount ranging between any combination of these values, inclusive of the recited values.

The alkylene oxides useful in alkoxylating the initiator to produce the inventive long-chain polyether polyols include, but are not limited to, ethylene oxide, propylene oxide, oxetane, 1 ,2- and 2,3-butylene oxide, isobutylene oxide, epichtorohydrin, cyclohexene oxide, styrene oxide, and the higher alkylene oxides such as the C₅- C₃₀ α-alkylene oxides. Propylene oxide alone or mixtures of propylene oxide with ethylene oxide or another alkylene oxide are preferred. Other polymerizable monomers may be used as well, e.g. anhydrides and other monomers as disclosed in U.S. Pat. Nos. 3,404,109, 3,538,043 and 5,145,883, the contents of which are herein incorporated in their entireties by reference thereto.

The inventive long-chain polyether polyols may preferably be reacted with a polyisocyanate, optionally in the presence of blowing agents, surfactants, cross-linking agents, extending agents, pigments, flame retardants, catalysts and fillers to produce flexible polyurethane foams.

Suitable polyisocyanates are known to those skilled in the art and include unmodified isocyanates, modified polyisocyanates, and isocyanate prepolymers. Such organic polyisocyanates include aliphatic, cycloaliphatic, araliphatic, aromatic, and heterocyclic polyisocyanates of the type described, for example, by W. Siefken in Justus Uebigs Annalen der Chemie, 562, pages 75 to 136. Examples of such lsocyanates include those represented by the formula

Q(NCO)ₙ

in which n is a number from 2-5, preferably 2-3, and Q is an aliphatic hydrocarbon group; a cycloaliphatic hydrocarbon group; an araliphatic hydrocarbon group; or an aromatic hydrocarbon group.

Examples of suitable isocyanates include ethylene diisocyanate; 1,4-tetramethylene diisocyanate; 1,6-hexamethylene diisocyanate; 1,12-dodecane diisocyanate; cyclobutane-1,3-diisocyanate; cyclohexane-1,3- and -1,4-diisocyanate, and mixtures of these isomers; 1-isocyanato-3,3,5-trimethyl-5-isocyanatomethylcyclohexane (isophorone diisocyanate;. German Auslegeschrift 1,202,785 and U.S. Pat. No. 3,401,190); 2,4- and 2,6-hexahydrotoluene diisocyanate and mixtures of these isomers; dicyclohexylmethane-4,4'-diisocyanate (hydrogenated MDI, or HMDI); 1,3-and 1,4-phenylene diisocyanate; 2,4- and 2,6-toluene diisocyanate and mixtures of these isomers (TDI); diphenylmethane-2,4'- and/or -4,4'-diisocyanate (MDI); polymeric diphenylmethane diisocyanate (PMDI), naphthylene-1,5-diisocyanate; triphenylmethane-4,4',4"-triisocyanate; polyphenyl-polymethylene-polyisocyanates of the type which may be obtained by condensing aniline with formaldehyde, followed by phosgenation (crude MDI), which are described, for example, in GB 878,430 and GB 848,671; norbomane diisocyanates, such as described in U.S. Pat. No. 3,492,330; m-and p-isocyanatophenyl sulfonylisocyanates of the type described in U.S. Pat. No. 3,454,606; perchlorinated aryl polyisocyanates of the type described, for example, in U.S. Pat. No. 3,227,138; modified polyisocyanates containing carbodiimide groups of the type described in U.S. Pat. No. 3,152,162; modified polyisocyanates containing urethane groups of the type described, for example, in U.S. Pat. Nos. 3,394,164 and 3,644,457; modified polyisocyanates containing allophanate groups of the type described, for example, in GB 994,890, BE 761,616, and NL 7,102,524; modified polyisocyanates containing isocyanurate groups of the type described, for example, in U.S. Pat. No. 3,002,973. German Patentschriften 1,022,789, 1,222,067 and 1,027,394, and German Offenlegungsschriften 1,919,034 and 2,004,048; modified polyisocyanates containing urea groups of the type described in German Patentschrift 1,230,778; polyisocyanates containing biuret groups of the type described, for example, in German Patentschrift 1,101,394, U.S. Pat. Nos. 3,124,605 and 3,201,372, and in GB 889,050; polyisocyanates obtained by telomerization reactions of the type described, for example, in U.S. Pat. No. 3,654,106; polyisocyanates containing ester groups of the type described, for example, in GB 965,474 and GB 1,072,956, in U.S. Pat. No. 3,567,763, and in German Patentschrift 1,231,688; reaction products of the above-mentioned isocyanates with acetals as described in German Patentschrift 1,072,385; and polyisocyanates containing polymeric fatty acid groups of the type described in U.S. Pat. No. 3,455,883. It is also possible to use the isocyanate-containing distillation residues accumulating in the production of isocyanates on a commercial scale, optionally in solution in one or more of the polyisocyanates mentioned above. Those skilled in the art will recognize that it is also possible to use mixtures of the polyisocyanates described above. Particularly preferred in the polyurethane foams of the present invention are 2,4- and 2,6-toluene diisocyanate and mixtures of these isomers (TDI).

Prepolymers may also be employed in the preparation of the inventive foams. Prepolymers may be prepared by reacting an excess of organic polyisocyanate or mixtures thereof with a minor amount of an active hydrogen-containing compound as determined by the well-known Zerewitinoff test, as described by Kohler in Journal of the American Chemical Society, 49, 3181(1927). These compounds and their methods of preparation are known to those skilled in the art. The use of any one specific active hydrogen compound is not critical; any such compound can be employed in the practice of the present invention.

Suitable additives optionally included in the polyurethane forming formulations of the present invention include, for example, stabilizers, catalysts, cell regulators, reaction inhibitors, plasticizers, fillers, crosslinking or extending agents, blowing agents, etc.

Stabilizers which may be considered suitable for the inventive foam forming process include, for example, polyether siloxanes, and preferably those which are insoluble in water. Compounds such as these are generally of such a structure that a relatively short chain copolymer of ethylene oxide and propylene oxide is attached to a polydimethylsiloxane residue. Such stabilizers are described in, for example, U.S. Pat. Nos. 2,834,748, 2,917,480 and 3,629,308.

Catalysts suitable for the foam forming process of the present invention include those which are known in the art. These catalysts include, for example, tertiary amines, such as triethylamine, tributytamine, N-methylmorpholine, N-ethylmorpholine, N,N,N',N'-tetramethylethylenediamine, pentamethyl-diethylenetriamine and higher homologues (as described in, for example, DE-A 2,624,527 and 2,624,528), 1,4-diazabicyclo(2.2.2)octane, N-methyl-N'-dimethyl-aminoethylpiperazine, bis-(dimethylaminoalkyl)piperazines, N,N-dimethylbenzylamine, N,N-dimethylcyclohexylamine, N,N-diethyl-benzylamine, bis-(N,N-diethylaminoethyl) adipate, N,N,N',N'-tetramethyl-1,3-butanediamine, N,N-dimethyt-β-phenylethylamine, 1,2-dimethylimidazole, 2-methylimidazole, monocyclic and bicyclic amines together with bis-(dialkylamino)alkyl ethers, such as 2,2-bis-(dimethylaminoethyl) ether.

Other suitable catalysts which may be used in producing the inventive polyurethane foams include, for example, organometallic compounds, and particularly, organotin compounds. Organotin compounds which may be considered suitable include those organotin compounds containing sulfur. Such catalysts include, for example, di-n-octyltin mercaptide. Other types of suitable organotin catalysts include, preferably tin(II) salts of carboxylic acids such as, for example, fin(II) acetate, tin(II) octoate, tin(II) ethylhexoate and/or tin(II) laurate, and tin(IV) compounds such as, for example, dibutylktin oxide, dibutyltin dichloride, dibutyltin diacetate, dibutyltin dilaurate, dibutyltin maleate and/or dioctyltin diacetate.

Water is preferably used as the sole blowing agent in the foams made according to the present invention, although auxiliary blowing agents, such as, for example, carbon dioxide, can be used. Water functions as the blowing by reacting with the isocyanate component to chemically form carbon dioxide gas plus an amine moiety which reacts further with the polyisocyanate to form urea backbone groups. Water can be used in an amount up to 10% by weight. Preferably, 1 to 8% by weight, more preferably, 1 to 5 % by weight, based on the total weight of the isocyanate-reactive mixture, of water is used in the present invention.

Further examples of suitable additives, which may optionally be included in the flexible polyurethane foams of the present invention can be found in Kunststoff-Handbuch, volume VII, edited by Vieweg & Hochtlen, Carl Hanser Verlag, Munich 1993, 3rd Ed., pp. 104 to 127, for example. The relevant details concerning the use and mode of action of these additives are set forth therein.

### EXAMPLES

The present invention is further illustrated, but is not to be limited, by the following examples. All quantities given in "parts" and "percents" are understood to be by weight, unless otherwise indicated. For the examples summarized below, the following materials were used:
- Polyol A:: a polyether polyol based on propoxylated glycerine having a hydroxyl number of 240 mg KOH/g;
- Polyol B:: a polyether polyol initiator based on propoxylated glycerine having a hydroxyl number of 350 mg KOH/g, contains 4 wt. % KOH;
- Polyol C:: a polyether polyol initiator based on propoxylated sorbitol having a hydroxyl number of 200 mg KOH/g, contains 2.2 wt. % KOH;
- Polyol D:: a 20 OH# glycerine initiated propylene oxide based trifunctional polymer polyol containing 43% styrene-acrylonitrile solids and a 20 wt. % ethylene oxide cap;
- Polyol E:: A 31.5 OH# glycerine/sorbitol (72:28 pbw) started propylene oxide based polyether polyol having 18 wt. % ethylene oxide cap;
- PEG-400:: a dihydroxy terminated 400 MW polyethylene glycol available commercially from Aldrich Chemical Company;
- TPEG-990:: a trihydroxy terminated 990 MW ethoxylated glycerine available commercially from Dow Chemical;
- DEOA:: diethanolamine;
- DC 5043:: a silicone surfactant available from Dow Coming;
- NtAX A 1:: bis(2-(Dimethylamino)ethyl)ether urethane catalysts available from OSi Specialties;
- NIAX A 33:: stannous octoate urethane catalyst available from OSi Specialties; and
- MONDUR TD-80:A: mixture of isomers 2,4- and 2,6-toluene diisocyanate available from Bayer MaterialScience LLC.

### Example C-1

In this comparative example, Polyol A (190 g) and 50 % aqueous KOH (4.74 g) were charged to a one-liter polyether polyol reactor. The mixture was stripped for 30 minutes under vacuum (-0.5 psia) with a nitrogen purge at 110°C to remove water. The nitrogen purge was stopped and vacuum valve to the reactor was closed, thus blocking the vacuum (0.5 psia) in the reactor. Propylene oxide (300 g) was fed to the reactor using a pressure feed back loop to control feed rate in order to maintain 50 psia pressure in the reactor throughout the process. The time required to add the propylene oxide was recorded and used to determine absolute feed rate (g/min).

### Examples 2-5

The following procedure was used; see Table I for details and charge weights for the individual examples. Polyol A. 50 % aqueous KOH (4.68 g) and either PEG-400 or TPEG-990 (see Table. I) were charged to a one-liters polyether polyol reactor. The mixture was stripped for 30 minutes under vacuum (-0.5 psia) with a nitrogen purge at 110°C to remove water. The nitrogen purge was stopped and vacuum valve to the reactor was closed, thus blocking the vacuum (0.5 psia) in the reactor. Propylene oxide (300 g) was fed to the reactor using a pressure feed back loop to control feed rate In order to maintain 50 psia pressure in the reactor throughout the process. The time required to add the propylene oxide was recorded and used to determine absolute feed rate (glmin).

**Table I**

| | **C-1** | **C-2** | **Ex. 3** | **C-4** | **Ex. 5** |
|---|---|---|---|---|---|
| Polyol A | 190 | 169 | 169 | 140 | 140 |
| PEG-400 (g) | - | 14 | | 43 | |
| TPEG-990 | | | 14 | | 43 |
| Propylene Oxide (g) | 300 | 300 | 300 | 300 | 300 |
| Linear Polyoxyethylene Content (wt. %) | - | 2.9 | - | 8.8 | - |
| Non-Linear Polyoxyethylene Content (wt. %) | - | - | 2.9 | - | 8.8 |
| PO Addition time (min.) | 211 | 180 | 197 | 140 | 147 |
| PO Addition Rate (g/min) | 1.43 | 1.67 | 1.52 | 2.14 | 2.04 |
| Relative Rate PO Addition | 1.0 | 1.17 | 1.06 | 1.50 | 1.43 |

The feed rates for the examples prepared with the non-linear polyoxyethylene-containing additives (Ex. 3 and 5) are shown along with Comparative Example C-1 (prepared without a polyoxyethylene-containing additive) in Table I. In addition, Comparative Examples C-2 and C-4 are shown, where a linear polyoxyethylene-containing additive was used at the same level as the non-linear polyoxyethylene-containing additive of the invention. As can be appreciated by reference to Table I, it was found that the rate of the KOH-catalyzed propoxylation reaction at 110°C could be accelerated by approximately 43% with incorporation of about 9 wt. % of the non-linear polyoxyethylene-containing additive, TPEG-990, and approximately 6% with about 3 wt. % TPEG-990. Although admittedly higher alkoxylation rates were achieved under the same conditions with the linear polyoxyethylene-containing additives (Comparative Examples G2 and C-4), the acceleration seen with the non-linear polyoxyethylene-containing additive of the invention is nonetheless significant.

Based on these results, the inventive concept was extended to the synthesis of an ethylene oxide-capped molded foam triol analogous to Polyol E, which was subsequently used to prepare a flexible polyurethane foam. The following examples demonstrate that the polyols prepared using a non-linear polyoxyethylene-containing additive can be used to prepare flexible polyurethane foams without detriment to the foam properties.

### Example C-6

In a five-gallon polyether polyol reactor, a start mixture was prepared from 60% Polyol B and 40% Polyol C. This start mixture was stripped under vacuum (-0.5 psia) at 105°C, while allowing nitrogen to flow through the reactor. After thirty minutes, the nitrogen feed was stopped, and the vacuum valve was closed, thus blocking the vacuum in the reactor. The mixture was propoxylated at 105°C to a hydroxyl number of 37 mg KOH/g. The propylene oxide was fed at a constant rate sufficient to give a seven-hour PO addition time. During the propoxylation, the reactor pressure was monitored, and the peak pressure was recorded. Following the propoxylation, the polyol was ethoxylated at (117°C) to a theoretical hydroxyl number of 31.5 mag KOH/g.

### Examples 7-8

An analogous procedure to that described for Comparative Example C-6 was used except a portion of the start mixture was replaced by TPEG-990, sufficient to give - 3 % of this non-linear polyoxyethylene-containing compound in the polyol prior to EO capping. The pressure observed during the propoxylation was recorded. Following the propoxylation, the long-chain polyols were ethoxylated in a procedure analogous to that used for Ex. C-6 to a hydroxyl number of 31.5 mg KOH/g.

As can be appreciated by reference to Table II, in the control with a seven-hour feed time (Ex. C-6), the pressure during the feed peaked at approximately 63 psia. As seen in Example 7, the addition of 3% TPEG-990. based on the weight of the propoxylate, gave a reduction in the peak pressure during the propylene oxide ("PO") addition to 47 psia, indicating a significantly higher reaction rate under these conditions. The feed time was decreased from seven to five hours (Ex. 8), with the resulting pressure being 55 psia, again consistent with the higher reactivity of the TPEG containing system. Analytical data for these triols are also presented in Table II. Hydroxyl number, viscosity, and unsaturation levels were within the normal specification range for the control product, prepared without the non-linear polyoxyethylene containing additive.

**Table II**

| | **C-6** | **Ex. 7** | **Ex. 8** |
|---|---|---|---|
| Polyol B | 1,403 | 1,109 | 1,127 |
| Polyol C | 943 | 745 | 756 |
| TPEG-990 | - | 579 | 588 |
| KOH added | - | 27.8 | 27.8 |
| KOH (wt. % in product) | 0.32 | 0.32 | 0.32 |
| peg-990 (wt. % in propoxylate)* | 0 | 3 | 3 |
| Feed time (min.) | 420 | 420 | 300 |
| Max. Press. (psia) | 63 | 47 | 55 |
| PO feed (g) | 18,439 | 17,062 | 17,329 |
| EO feed (g) | 3,959 | 3,713 | 3,771 |
| OH# (mg KOH/g) | 31.1 | 32.8 | 31.7 |
| Viscosity (cSt) | 1092 | 1003 | 988 |
| Unsaturation (meq./g) | 0.029 | 0.038 | 0.046 |

| | | | |
|---|---|---|---|
| *Corresponds to weight % in the polyol prior to EO capping | | | |

### Examples 9-12

Molded polyurethane flexible foams were prepared using the formulations shown in Table III. The foams were prepared by first combining and thoroughly mixing all of the indicated ingredients except the MONDUR TD-80 to produced a polyol blend. This polyol blend was then combined with the MONDUR TD-80 and mixed with a mechanical mixer for 30 seconds. The mixture was poured into a pre-heated mold (150 F), to produce a test block having a density of 1.9 pounds/ft³. After 4.5 minutes, the foams were demolded, and immediately the force required to crush the foams on the 1^{st}, 3d, and 7^{th} crush cycle was measured. The foams were aged for 1 week prior to determining the mechanical properties (see Table. III).

As is apparent from the data in Tabte III, using polyols prepared according to the invention to produce molded foams (Ex. 11 and Ex. 12) gave foams with mechanical properties quite similar to Comparative Examples (C-9 and C-10), which were prepared using a standard commercial molded polyol (e.g. Polyol E) or a laboratory prepared analogue of Polyol E; (Ex. C-6).

**Table III**

| **Formulation Component** | **Ex. C-9** | **Ex. C-10** | **Ex. 11** | **Ex. 12** |
|---|---|---|---|---|
| Polyol D | 15 | 15 | 15 | 15 |
| Polyol E | 85 | | | |
| Polyol of Ex. C-6 | | 85 | | |
| Polyol of Ex. 7 | | | 85 | |
| Polyol of Ex. 8 | | | | 85 |
| Water | 4.26 | 4.26 | 4.26 | 4.26 |
| DEOA | 1.2 | 1.2 | 1.2 | 1.2 |
| DC 5043 | 1 | 1 | 1 | 1 |
| NIAX A-1 | 0.08 | 0.08 | 0.08 | 0.08 |
| NIAX A-33 | 0.32 | 0.32 | 0.32 | 0.32 |
| MONDUR TD-80 | 49.1 | 49.1 | 49.1 | 49.1 |
| | | | | |
| **Physical Property** | | | | |
| Force to Crush (lb.) (1^{st} 3d, 7^{th} cycle) | 250-122-44 | 273 -157-63 | 240-133-54 | 253-137-51 |
| Free Rise settle (%) | 5.43 | 4.92 | 3.49 | 4.15 |
| Cell Structure | normal | normal | normal | normal |
| Density (lb/ft³) | 1.95 | 1.97 | 1.90 | 2.08 |
| Resilience (%) | 66.3 | 66.7 | 64.3 | 58.0 |
| Air Flow (ft³/min) | 3.48 | 3.53 | 2.56 | 3.48 |
| IFD 10% (lb/50in²) | 16.2 | 14.5 | 14.8 | - |
| IFD 25% (lb/50in²) | 25.7 | 23.2 | 23.7 | 22.3 |
| IFD 50% (lb/50in²) | 45.9 | 42.1 | 43.2 | 40.9 |
| IFD 25% Return | 21.5 | 19.7 | 19.6 | 17.3 |
| CFD 50% (psi) | 0.21 | 0.18 | 0.20 | 0.22 |
| Tensile Strength (psi) | 15.4 | 16.0 | 15.6 | 15.5 |
| Elongation (%) | 123.0 | 120.6 | 121.9 | 112 |
| Tear Strength (pli) | 1.15 | 1.03 | 1.19 | 1.17 |
| Comp. Set. 50% (%) | 8.7 | 11.0 | 12.1 | 11.6 |
| Humid Ages (50% RH) Comp. Set (%) | 17.4 | 23.8 | 22.3 | 21.9 |

The foregoing examples of the present invention are offered for the purpose of illustration and not limitation. It will be apparent to those skilled in the art that the embodiments described herein may be modified or revised in various ways without departing from the spirit and scope of the invention. The scope of the invention is to be measured by the appended claims.

## Claims

1. A process for producing a long chain polyether polyol composing:
alkoxylating an initiator with an alkylene oxide which is selected from the group consisting of (i) propylene oxide and (ii) a block of propylene oxide, followed by a block of ethylene oxide, in the presence of a basic catalyst having at least one cation thereof cheated with 2 wt.% to 9 wt.% of a non-linear polyoxyethylene-containing compound having a molecular weight of 300 to 1,000 g/mole and a functionality of at least 3, wherein the long chain polyether polyol has a number average molecular weight of more than 1,200 g/mole.

2. The process according to Claim 1, wherein the long chain polyether polyol has a number average molecular weight of from 1,200 g/mole to 50,000 g/mole.

3. The process according to Claim 1, wherein the long chain polyether polyol has a number average molecular weight of from 1,200 g/mole to 30,000 g/mole.

4. The process according to Claim 1, wherein the long chain polyether polyol has a number average molecular weight of from 1,200 g/mole to 8,000 g/mole.

5. The process according to Claim 1, wherein the initiator is chosen from C₁-C₃₀ monols, ethylene glycol, diethylene glycol, triethylene glycol, propylene glycol, 1,3-propanediol, dipropylene glycol, tripropylene glycol, neopentyl glycol, 1,4-butanediol, 1,3-butanediol, 1,2-butanediol, 2,3-butanediol, 1,6-hexanediol, glycerin, trimethylolpropane, trimethylolethane, pentaerythritol, α-methylglucoside, sorbitol, mannitol, hydroxymethylglucoside, hydroxypropylglucoside, sucrose, N,N,N',N'-tetrakis[2-hydroxyethyl or 2-hydroxypropyl]ethylene diamine, 1,4-cyclohexanediol, cyclohexanedimethanol, hydroquinone, resorcinol, and mixtures thereof.

6. The process according to Claim 1, wherein the basic catalyst is chosen from potassium hydroxide, sodium hydroxide, barium hydroxide and cesium hydroxide.

7. The process according to Claim 1, wherein the basic catalyst is potassium hydroxide.

8. The process according to Claim 1, wherein the non-linear polyoxyethylene-containing compound has a functionality of from greater than 3 to 8.

## Patentansprüche

1. Verfahren zur Herstellung eines langkettigen Polyetherpolyols, umfassend: das Alkoxylieren eines Initiators mit einem Alkylenoxid, das aus der aus (i) Propylenoxid und (ii) einem Propylenoxidblock gefolgt von einem Ethylenoxidblock bestehenden Gruppe ausgewählt ist, in Gegenwart eines basischen Katalysators, der zumindest ein Kation aufweist, das mit 2 Gew.-% bis 9 Gew.-% einer unverzweigten polyoxyethylenhältigen Verbindung mit einem Molekulargewicht von 300 bis 1.000 g/mol und einer Funktionalität von zumindest 3 chelatisiert ist, worin das langkettige Polyetherpolyol ein zahlenmittleres Molekulargewicht von mehr als 1.200 g/mol aufweist.

2. Verfahren nach Anspruch 1, worin das langkettige Polyetherpolyol ein zahlenmittleres Molekulargewicht von 1.200 g/mol bis 50.000 g/mol aufweist.

3. Verfahren nach Anspruch 1, worin das langkettige Polyetherpolyol ein zahlenmittleres Molekulargewicht von 1.200 g/mol bis 30.000 g/mol aufweist.

4. Verfahren nach Anspruch 1, worin das langkettige Polyetherpolyol ein zahlenmittleres Molekulargewicht von 1.200 g/mol bis 8.000 g/mol aufweist.

5. Verfahren nach Anspruch 1, worin der Initiator aus Folgendem ausgewählt ist: C₁₋₃₀-Monole, Ethylenglykol, Diethylenglykol, Triethylenglykol, Propylenglykol, 1,3-Propandiol, Dipropylenglykol, Tripropylenglykol, Neopentylglykol, 1,4-Butandiol, 1,3-Butandiol, 1,2-Butandiol, 2,3-Butandiol, 1,6-Hexandiol, Glycerin, Trimethylolpropan, Trimethylolethan, Pentaerythrit, α-Methylglucosid, Sorbit, Mannit, Hydroxymethylglucosid, Hydroxypropylglucosid, Saccharose, N,N,N',N'-Tetrakis[2-hydroxyethyl oder 2-hydroxypropyl]ethylendiamin, 1,4-Cyclohexandiol, Cyclohexandimethanol, Hydrochinon, Resorcinol und Gemischen davon.

6. Verfahren nach Anspruch 1, worin der basische Katalysator aus Kaliumhydroxid, Natriumhydroxid, Bariumhydroxid und Cäsiumhydroxid ausgewählt ist.

7. Verfahren nach Anspruch 1, worin der basische Katalysator Kaliumhydroxid ist.

8. Verfahren nach Anspruch 1, worin die unverzweigte polyoxyethylenhältige Verbindung eine Funktionalität von mehr als 3 bis 8 aufweist.

## Revendications

1. Procédé de production d'un polyétherpolyol à chaîne longue comprenant :
l'alcoxylation d'un initiateur avec un oxyde d'alkylène qui est choisi dans le groupe constitué de (i) l'oxyde de propylène et (ii) une séquence d'oxyde de propylène, suivie par une séquence d'oxyde d'éthylène, en présence d'un catalyseur basique présentant au moins un cation de celui-ci chélaté avec de 2 % en poids à 9 % en poids d'un composé non linéaire contenant du polyoxyéthylène ayant un poids moléculaire de 300 à 1 000 g/mol et une fonctionnalité d'au moins 3, dans lequel le polyétherpolyol à chaîne longue présente un poids moléculaire moyen en nombre supérieur à 1 200 g/mol.

2. Procédé selon la revendication 1, dans lequel le polyétherpolyol à chaîne longue présente un poids moléculaire moyen en nombre de 1 200 g/mol à 50 000 g/mol.

3. Procédé selon la revendication 1, dans lequel le polyétherpolyol à chaîne longue présente un poids moléculaire moyen en nombre de 1 200 g/mol à 30 000 g/mol.

4. Procédé selon la revendication 1, dans lequel le polyétherpolyol à chaîne longue présente un poids moléculaire moyen en nombre de 1 200 g/mol à 8 000 g/mol.

5. Procédé selon la revendication 1, dans lequel l'initiateur est choisi parmi des monols en C₁-C₃₀, l'éthylèneglycol, le diéthylèneglycol, le triéthylèneglycol, le propylèneglycol, le 1,3-propanediol, le dipropylèneglycol, le tripropylèneglycol, le néopentylglycol, le 1,4-butanediol, le 1,3-butanediol, le 1,2-butanediol, le 2,3-butanediol, le 1,6-hexanediol, la glycérine, le triméthylolpropane, le triméthyloléthane, le pentaérythritol, l'α-méthylglucoside, le sorbitol, le mannitol, l'hydroxyméthylglucoside, l'hydroxypropylglucoside, le saccharose, la N,N,N',N'-tétrakis[2-hydroxyéthyl ou 2-hydroxypropyl]éthylènediamine, le 1,4-cyclohexanediol, le cyclohexanediméthanol, l'hydroquinone, le résorcinol et des mélanges de ceux-ci.

6. Procédé selon la revendication 1, dans lequel le catalyseur basique est choisi parmi l'hydroxyde de potassium, l'hydroxyde de sodium, l'hydroxyde de baryum et l'hydroxyde de césium.

7. Procédé selon la revendication 1, dans lequel le catalyseur basique est l'hydroxyde de potassium.

8. Procédé selon la revendication 1, dans lequel le composé non linéaire contenant du polyoxyéthylène présente une fonctionnalité de 3 à 8.
